# EUROPEAN PATENT APPLICATION

(11) **EP 4 147 761 A1**
(43) Date of publication of application: **15.03.2023**
(21) Application number: 22197320.9
(22) Date of filing: 30.04.2015
(51) Int. Cl.: B01D 15/20, B01D 15/38, C07K 16/06, B01D 15/12, B01D 15/18, B01D 15/36, B01D 15/42, C07K 1/22, C07K 16/00

(54) **METHODS FOR THE PURIFICATION OF PROTEINS USING CAPRYLIC ACID**

(30) Priority: 30.04.2014 EP 14166535
(62) Divisional of application: 20217541.0
(71) Applicant: Novo Nordisk A/S, 2880 Bagsvaerd (DK)
(72) Inventor: JENSEN, Ole Elvang, 2880 Bagsværd (DK); KÆRSGAARD, Per, 2880 Bagsværd (DK)

(57) **Abstract**

A protein purification process with virus inactivation or removal uses caprylic acid (octanoic acid) at acidic pH. The method comprises caprylic acid treatment as part of the chromatographic step so as to perform viral inactivation without a discontinuous process and without the requirement of attention by personnel, particularly when the pH adjustment of the eluate is performed automatically by eluting into buffer. The method of the invention further results in mycoplasmas being inactivated, and reduced impurities like Host Cell Protein (HCP).

## Description

### TECHNICAL FIELD

Protein purification with virus inactivation or removal is performed using caprylic acid.

### BACKGROUND

Biopharmaceuticals or the use of pharmaceutical compositions comprising a therapeutic protein for the treatment of diseases, disorders, or conditions is a strategic element for a number of pharmaceutical and biotechnology companies. Many proteins used in a biopharmaceutical composition are obtained through recombinant production using a mammalian cell line or by purification from a biological fluid. However, a therapeutic protein manufactured by such methods may be contaminated with a virus, ostensibly with a contagious pathogenic virus, which can be deleterious to the health of an individual. It is necessary to process a medium comprising a therapeutic protein in order to eliminate any potential contaminating viral activity.

The inactivation of contagious pathogenic viruses can be performed by heat-inactivation, solvent/detergent (S/D) inactivation, pH inactivation, chemical inactivation, and/or irradiation inactivation, with S/D inactivation perhaps the most widely accepted veridical method.

Most human pathogens are enveloped viruses. These are susceptible to membrane disruption by solvents and detergents. Inactivation methods using heat, acidic pH, chemicals and irradiation are problematic as these agents are harsh and/or invasive and tend to denature or otherwise inactivate the therapeutic protein being purified.

For the inactivation of viruses, as part of the virus clearance during the purification of a pharmaceutical product, the enveloped virus is usually inactivated by a detergent or low pH treatment step in which a solution of the pharmaceutical product is added detergent or adjusted to pH around 3.7 (3.5 - 3.9) for up to 6 hours or more. However, detergents are not always effective towards all kind of enveloped virus, they may have environmental problems or, for low pH inactivation, the window of operation is very narrow because at pH higher than 3.8, the inactivation effect wears off. Conversely, at pH values below 3.5 the risk of aggregate formation or denaturation increases dramatically for many proteins.

EP 0 374 625 has shown caprylic acid to be effective towards most enveloped virus in a short time. US 4,939,176 reports a process for inactivating viruses in solutions of biologically active proteins by contacting the solutions with caprylic acid. The preferred conditions recited for the process were pH 4 to pH 8, and 0.07 (%w/w) to 0.001 (%w/w) of the non-ionized form of caprylic acid.

Other methods of viral inactivation through the use of chemical agents are known. US 4,540,573 teaches the use of di- or tri-alkyl phosphates as antiviral agents. US 4,534,972 describes a method of rendering solutions of therapeutically or immunologically active proteins substantially free of infectious agents. In US 4,534,972, a solution of protein is contacted with a transition metal complex, e.g. copper phenanthroline, and a reducing agent to effect inactivation of viruses without substantially affecting the activity of the protein.

US 5,164,487 concerns the use of caprylic acid for the manufacture of an IgG preparation free from aggregates, vasoactive substances and proteolytic enzymes. The method includes contacting the starting material containing IgG with 0.4% to 1.5% caprylic acid before chromatographic purification with an ion exchange or hydrophobic matrix.

Steinbuch et al. showed the use of caprylic acid to precipitate most proteins and lipoproteins (other than the immunoglobulins) present in Cohn ethanol Fraction III. (Steinbuch et al., 1973). A two-step purification of immunoglobulins from mammalian sera and ascites fluid has been described (McKinney et al., 1987). First albumin and other non-IgG proteins were precipitated using caprylic acid, and then ammonium sulfate was added to the supernatant to precipitate the IgG.

During human immunoglobulin preparation caprylic acid is generally recognized as an effective precipitating agent for most plasma proteins at pH 4.8, so long as parameters such as temperature and ionic strength are optimized. Steinbuch et al. (1969) have described the precipitation of the bulk of the plasma proteins with caprylic acid without affecting IgG, ceruloplasmin and IgA. Steinbuch et al. isolated IgG from mammalian sera using caprylic acid and reported that extensive non-immunoglobulin precipitation was best obtained at slightly acidic pH, but not below pH 4.5. Plasma was diluted 2:1 with 0.06 M acetate buffer, pH 4.8, and then treated with 2.5 wt.% caprylate to initiate precipitation.

### SUMMARY

The present invention provides a method for the purification of a target protein from a sample comprising the steps of
i. loading a protein-containing sample onto a stationary phase, so as to bind the target protein to said stationary phase;
ii. subjecting the solid phase with the bound target protein to a caprylic acid solution; wherein the caprylic acid solution is at a pH so as to form a free caprylic acid, and wherein the caprylic acid solution is a caprylic acid buffer at a concentration of 1 to 50 mM, as measured in terms of free caprylic acid; and
iii. eluting the target protein

The present invention has finally managed to incorporate caprylic acid treatment as part of the chromatographic step so as to to perform viral inactivation without a discontinuous process and without the requirement of attention by personnel, particularly when the pH adjustment of the eluate is performed automatically by eluting into buffer.

An aspect of the invention is directed to a method for the purification of a target protein from a sample comprising the steps of:
i. loading a protein-containing sample onto a stationary phase, so as to bind the target protein to said solid phase;
ii. subjecting the solid phase to a caprylic acid solution; and
iii. eluting the target protein.

A further aspect of the invention is directed to a method for inactivation of a virus in a protein-containing mixture comprising steps of i. loading the sample onto a stationary phase, so as to bind the target protein to said solid phase; ii. subjecting the solid phase to a caprylic acid solution so as to inactivate and wash away the vira; and iii. eluting the target protein.

Another aspect of the invention is directed to a method for the preparation of a virus-free protein solution comprising the steps of i. loading a sample onto a stationary phase, so as to bind the target protein to said solid phase; ii. subjecting the solid phase to a caprylic acid solution wash so as to inactivate and wash away the vira; and iii. eluting the target protein.

An alternative aspect of the invention is directed to a viral inactivation method wherein a sample comprising a) a target protein and b) one or more virus types is loaded onto a stationary phase and washed with a caprylic acid solution.

A method of inactivating a lipid-coat containing virus, the method comprising the steps of: i. loading a sample onto a stationary phase, so as to bind the target protein to said solid phase; ii. subjecting the solid phase to a caprylic acid solution wash so as to inactivate and wash away the vira; and iii. eluting the target protein.

The invention is furthermore directed to a protein sample essentially free of a lipid-coat containing virus obtained from a method comprising the steps of: i. loading a sample onto a stationary phase, so as to bind the target protein to said solid phase; ii. subjecting the solid phase to a caprylic acid solution wash so as to inactivate and wash away the vira; and iii. eluting the target protein.

A further aspect of the invention is directed to method for inactivation of mycoplasmas in a protein-containing mixture comprising steps of i. loading the sample onto a stationary phase, so as to bind the target protein to said solid phase; ii. subjecting the solid phase to a caprylic acid solution so as to inactivate and wash away the mycoplasma; and iii. eluting the target protein.

The invention may also solve further problems that will be apparent from the disclosure of the exemplary embodiments.

### DESCRIPTION

An aspect of the invention is directed to concomitant viral inactivation and protein purification during a chromatographic step. By the method of the invention removes the need for participation by a technician for an inactivation step and separate loading onto a column. The singular inactivation and capture step can be performed without the participation of a technician.

Typically, purification of a target protein comprises inactivation of potential virus in said sample. Accordingly, an aspect of the invention is directed to the preparation of a virus-free protein solution and to a virus-free protein solution prepared according to the method of the present invention. The method of the invention involves the inactivation of virus by washing a chromatography column or membrane with a caprylic acid-containing washing solvent after load of protein-containing solution and prior to elution of the protein. The method of the invention also inactivates mycoplasmas and result in a higher reduction of Host Cell Protein (HCP).

For the protein purification and vira inactivation of the invention, the caprylic acid solution must comprise caprylic acid in its free or non-ionized acid; that is to say substantially as free caprylic acid or non-ionized caprylic acid. The terms "free caprylic acid" and "non-ionized caprylic acid" are intended to mean caprylic acid in its un-dissociated form; that is to say as in its protonated form; that is to say as CH₃(CH₂)₆CO₂H.

The invention is directed to the separation or purification of one or more target proteins, typically one target protein, from any medium or sample comprising the protein which may further comprise, potentially comprises or comprises a virus or vira. The sample may be a crude media or one that has been partially washed, filtered, diafiltered, or partially purified in any manner. Suitably, the sample is selected from the group consisting of a fermentation broth, a cell culture, cell line media, ascites fluid, tissue culture media, a transgenic cell medium, human or animal blood, human or animal plasma including plasma fractions. Typically, the sample is selected from cell line media, such as a transgenic cell medium and human or animal plasma.

The sample or protein-containing mixture is a fluid comprising a protein, typically a protein having a biological or therapeutic activity. A fluid may be any fluid having the possibility of being contaminated by a virus. Non-limiting examples of a fluid include tissue and cell culture extract like a cell lysate, a cell supernatant, placental extracts, or an ascites fluid; a biological fluid like blood, plasma, serum, milk, saliva, semen; or any other fluid that includes a protein having an activity, or any purified, partially purified for thereof, or crude liquid or colloid including an elution from a previous purification step.

As stated, the sample used in the invention has as it source cell line media, such as a transgenic cell medium and human or animal blood plasma.

A sample of the invention comprising the protein of the invention may be obtained from an organism that naturally expresses the protein, from a transgenic organism genetically-engineered to express the protein, or from a cell line recombinantly producing the protein. Non-limiting examples of a transgenic organisms include organisms disclosed herein that have been genetically-engineered to express a protein of interest. A sample of the invention comprising the protein may be from an organism or transgenic organism may be obtained from a biological fluid, tissue or organ extract, or other source from an organism using routine methods known in the art. Furthermore, various prokaryote and/or eukaryotic expression systems may also be a source of the sample of the invention, such as to recombinantly express a protein disclosed herein. Expression systems as a source of the protein of the invention can include, without limitation, inducible expression, non-inducible expression, constitutive expression, tissue-specific expression, cell-specific expression, viral-mediated expression, stably- integrated expression, and transient expression.

A protein of the invention may be encoded by a polynucleotide cloned into an expression vector. Prokaryote expression vectors, eukaryotic expression vectors, yeast vectors, insect vectors, mammalian vectors and other suitable expression vectors are known in the art and commercially available.

After the initial harvesting from an organism, transgenic organism, or cell culture system, a sample comprising the protein of the invention may undergo a purification or filtration process, including a diafiltration process. This initial or preliminary purification of the sample may include one or more of concentration of the protein in the sample, intermediate purification steps to remove impurities, and polishing to remove additional impurities and protein variants.

An object of the invention is the inactivation of virus in a protein-containing sample. The phrase "viral inactivation" is intended to mean a decrease in the number of viral particles in a particular sample ("reduction"), and/or a decrease in the activity, such as, but not limited to, the infectivity and/or the ability to replicate, of viral particles in a particular sample. The term "viral inactivation" means a virion, normally capable of replication, is rendered unable to replicate. The term further encompasses removal, at least in part, or decrease or reduction in the number, of the virus from the sample. The term encompasses performing the method where the presence or absence of virus is unknown. For example, the process may be applied to proteins from whole blood or blood plasma or cell supernatant in which the presence or absence of virus is not characterized and there is no assurance that the proteins from plasma and cell supernatant is safe for administration to an individual. Embodiments of the present invention result in, but not limited to, more than six log₁₀ reduction in viral load. That is, the method produces a reduction in viral replication compared to control of 1,000,000, based on current detection limits.

Said decreases in the number and/or activity of viral particles can be on the order of about 1% to about 100%, preferably of about 20% to about 100%, more preferably of about 30% to about 100%, more preferably of about 40% to about 100%, even more preferably of about 50% to about 100%, even more preferably of about 60% to about 100%, yet more preferably of about at least about 70%, such as at least about 80%, more typically at least about 90%, such as from about 95% to 100%, preferably from about 95% to about 100%, typically from about 99 to about 100%.

In certain non-limiting embodiments, the total amount of virus, if any, in the purified antibody product is less than the ID50 (the amount of virus that will infect 50 percent of a target population) or PFU (plaque forming units) for that virus, preferably at least 100-fold less than the ID50/PFU for that virus, more preferably at least 10,000-fold less than the ID50/PFU for that virus, and still more preferably at least 1,000,000-fold less than the ID50/PFU for that virus.

As will be recognised by the person skilled in the art, the method of the invention can either inactivate viruses in the sample which can be later removed from the sample or the method can both inactivate and remove viruses from the sample.

Aspects of the present specification disclose, in part, a lipid-coat containing virus. A complete virus particle, known as a virion, consists of nucleic acid, either DNA or RNA, surrounded by a protective coat of protein called a capsid. Viruses can be grouped into non-enveloped and enveloped viruses. As used herein, the term "lipid-coat containing virus" refers to any virus comprising a membrane or envelope including lipid, such as, e.g., an enveloped virus. Enveloped viruses have their capsid enclosed by a lipoprotein membrane, or envelope. This envelope is derived from the host cell as the virus "buds" from its surface and consists mostly of lipids not encoded by the viral genome. Enveloped viruses range in size from about 45-55 nm to about 120-200 nm. Lipid-coat containing viruses which can infect mammalian cells include DNA viruses like a herpesviridae virus, a poxviridae virus, or a hepadnaviridae virus; RNA viruses like a flaviviridae virus, a togaviridae virus, a coronaviridae virus, a deltavirus virus, an orthomyxoviridae virus, a paramyxoviridae virus, a rhabdoviridae virus, a bunyaviridae virus, or a filoviridae virus; and reverse transcribing viruses like a retroviridae virus or a hepadnaviridae virus. Non- limiting examples of lipid-coat containing viruses include a human immunodeficiency virus, a sindbis virus, a herpes simplex virus, a pseudorabies virus, a sendai virus, a vesicular stomatitis virus, a West Nile virus, a bovine viral diarrhea virus, a corona virus, an equine arthritis virus, a severe acute respiratory syndrome virus, a murine leukemia virus (MuLV) , an Infectious Bovine Rhinotracheitits virus (IBRV), or a vaccinia virus.

A non-enveloped virus refers to a virus whose capsid lacks a lipoprotein membrane, or envelope. In a non-enveloped virus, the capsid mediates attachment to and penetration into host cells. Capsids are generally either helical or icosahedral. Non-enveloped viruses range in size from about 18-26 nm to about 70-90 nm. Non-enveloped viruses which can infect mammalian cells include, e.g., a parvoviridae virus, an adenoviridae virus, a birnaviridae virus, a papillomaviridae virus, a polyomaviridae virus, a picornaviridae virus, a reoviridae virus, and a calciviridae virus.

The virus may be, without limitation, MuLV, IBRV, HIV, hepatitis B, hepatitis C, Ebola, West Nile and hantavirus, have the potential to contaminate blood supplies and blood based products. It is difficult to remove or inactivate viral agents potentially present in blood products without severely compromising the quality and/or the functionality of the proteins. Non-limiting examples of industrially relevant viruses are: parvoviradae, paramyoxyiradae, orthomyxoviradae, bunyaviridae, rhabdoviridae, reoviridae, togaviridae, caliciviridae, Reoviridae, and picornaviridae.

The virus includes, but is not limited to, a single-stranded DNA virus, a double-stranded DNA virus, a double-stranded RNA virus, and a single-stranded RNA virus. In further aspects, the virus is an enveloped virus. Enveloped viruses includes, but is not limited to, a retrovirus, a herpes virus, or a hepadnavirus. In any variation of the invention, a virus may be selected from the group consisting of an adenovirus, African swine fever-line virus, arenavirus, arterivirus, astrovirus, baculovirus, badnavirus, barnavirus, birnavirus, bromovirus, bunyavirus, calicivirus, capillovirus, carlavirus, caulimovirus, circovirus, closterovirus, comovirus, coronavirus, cotricovirus, cystovirus, deltavirus, dianthovirus, enamovirus, filovirus, flavivirus, furovirus, fusellovirus, geminivirus, hepadnavirus, herpesvirus, hordeivirus, hypovirus, ideaovirus, inovirus, iridovirus, levivirus, lipothrixvirus, luteovirus, machlomovirus, marafivovirus, microvirus, myovirus, necrovirus, nodavirus, orthomyxovirus, papovavirus, paramyxovirus, partitivirus, parvovirus, phycodnavirus, picornavirus, plamavirus, podovirus, polydnavirus, potexvirus, potyvirus, poxvirus, reovirus, retrovirus, rhabdovirus, rhizidiovirus, sequevirus, siphovirus, sobemovirus, tectivirus, tenuivirus, tetravirus, tobamavirus, tobravirus, togavirus, tombusvirus, totivirus, trichovirus, tymovirus, and umbravirus. In some aspects, viruses include, but are not limited to, epizootic hemorrhagic disease virus (EHDV), mice minute virus (MMV), mouse parvovirus-1 (MPV), cache valley virus, Vesivirus 2117, porcine circovirus (PCV 1), porcine circovirus 2 (PCV 2), canine parvovirus (CPV), bovine parvovirus (BPV), or blue tongue virus (BTV).

A protein of the invention is typically a protein having an activity; preferably a biological activity. The protein may be any protein of interest in which elimination of any contaminating viral activity is desired.

The method of the invention is applicable to all proteins, without limitation. As known to the skilled practitioner, the protein in the protein-containing sample typically is a determinant in the selection of the stationary phase and thereby not a limiting factor in the method of the invention. The term protein is intended to mean an oligopeptide or polypeptide chain having a secondary and optionally a tertiary structure. The term protein includes oligopeptides, polypeptides and proteins that are glycosylated including glycoproteins or linked to nucleic acid sequences. Proteins may comprise any post-translation modification known to the person skilled in the art. Proteins may comprise non-proteinaceous elements, linkages or modifications. Particularly in relation to, but not limited to, proteins from transgenic cells, the proteins of the method of the invention may comprise one or several non-natural amino acids and/or non-natural isomers.

The method of the invention is applicable to proteins from all sources, without limitation. The protein in the protein-containing sample may be selected from a blood protein, a milk protein, a cellular or cellular extract protein. The protein in the protein-containing is typically a blood protein or protein from a cellular extract, or a hormone. More typically selected from the group consisting of an antibody, or a fragment thereof, such as a Fab or a single-chain antibody, a receptor such as a soluble receptor, a coagulation factor, such as FV, FVII, FVIII, FIX, FX, FXI, and FVIII, or a growth hormone.

The terms "polypeptide" and "protein" may be used interchangeably to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

The term "antibody" or "antibodies" is used in the broadest sense and specifically covers, for example, single monoclonal antibodies (including agonist, antagonist, and neutralizing antibodies), antibody compositions with polyepitopic specificity, polyclonal antibodies, single chain antibodies, multispecific antibodies (e.g., bispecific antibodies), immunoadhesins, and fragments of antibodies as long as they exhibit the desired biological or immunological activity. The term "immunoglobulin" (Ig) is used interchangeable with antibody herein.

The term "antibody fragments" comprise a portion of a full length antibody, generally the antigen binding or variable region thereof. Examples of antibody fragments include Fab, Fab', F(ab')2, and Fv fragments; single-chain antibody molecules; diabodies; linear antibodies; and multispecific antibodies formed from antibody fragments.

The term "monoclonal antibody" as used herein refers to an antibody obtained from a population of substantially homogeneous antibodies, i.e., the individual antibodies comprising the population are identical except for possible naturally occurring mutations that may be present in minor amounts. The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit biological activity.

As stated, accurate tailoring of the purification relies on consideration of the protein to be purified. In certain embodiments, the separation steps of the instant invention separate an antibody from one or more HCPs. Antibodies that can be successfully purified using the methods described herein include, but are not limited to, human IgA1, IgA2, IgD, IgE, IgG1 IgG2, IgG3, IgG4, and IgM antibodies.

In certain embodiments, the purification strategies of the instant invention exclude the use of Protein A affinity chromatography, for example purification of IgG3 antibodies, as IgG3 antibodies bind to Protein A inefficiently. Other factors that allow for specific tailoring of a purification scheme include, but are not limited to: the presence or absence of an Fc region (e.g., in the context of full length antibody as compared to an Fab fragment thereof) because Protein A binds to the Fc region; the particular germline sequences employed in generating to antibody of interest; and the amino acid composition of the antibody (e.g., the primary sequence of the antibody as well as the overall charge/hydrophobicity of the molecule). Antibodies sharing one or more characteristic can be purified using purification strategies tailored to take advantage of that characteristic.

A protein of the invention can be a blood protein such as a blood coagulation protein, albumin, and/or an immunoglobulin. Non-limiting examples of a blood protein include ADAMTS-13, a1-antiplasmin, a2-antiplasmin, antithrombin, antithrombin III, cancer procoagulant, erythropoietin, Factor II, Factor V, Factor VI, Factor VII, Factor VIII, Factor IX, Factor X, Factor XI, Factor XII, Factor XIII, fibronectin, fibrinogen, heparin cofactor II, high-molecular-weight kininogen, intramuscular immunoglobulin, intravenous immunoglobulin, plasminogen, plasminogen activator inhibitor-1 , plasminogen activator inhibitor-2, prekallikrein, protein C, protein S, protein Z, protein Z-related protease inhibitor, tissue factor, tissue plasminogen activator, urokinase, or Von Willebrand Factor.

The present invention is suitable for isolating and purifying antibodies from a sample. Typically, the sample comprises a cell line harvest wherein the cell line is employed to produce specific proteins, such as specific antibodies of the present invention.

Examples 2 and 3, taken together, demonstrate that the method of the invention allows for protein purification of different kinds of protein in that complete clearance of a virus, such as eMuLV, was achieved with concomitant purification of two different protein types, namely a recombinant human antibody (lgG1) in one instance and a Fab in another instance. Furthermore, the Examples demonstrate the concomitant purification of an antibody with viral inactivation.

In a preferred embodiment, the sample comprises a protein selected from an antibody, or a fragment thereof, a coagulation factor, and a hormone. The antibody is preferably selected from the group consisting of an anti-TNF antibody, an anti-IL-6 antibody, anti-IL-8 antibody, anti-IL-12 antibody, anti-IL-16 antibody, anti-IL-20 antibody, anti-IL-21 antibody, anti-IL-23 antibody, an anti-DR3 antibody, anti-CD27 antibody, an anti-C5aR-215 antibody, anti-CD30 antibody, anti-TREM-1 antibody, anti-TREM-2 antibody, anti-uPA antibody, anti-uPAR antibody, anti-OX40 antibody, anti-BTLA antibody, anti-GLP-1 antibody, anti-LAIR1 antibody, anti-LAIR2 antibody, anti-LILRA1 antibody, anti-Ly9 antibody, anti-PD1 antibody, anti-Siglec-9 antibody, anti-MMP2 antibody, anti-MMP6 antibody, anti-MMP8 antibody, anti-MMP9 antibody and anti-TIM3 antibody.

The protein may be a hormone such as a hormone selected from hGH, glucagon, kisspeptin and insulin.

In a typical embodiment, a column packed with a resin is loaded with clarified culture broth containing the target protein. The column is typically loaded to approximately 1 to 50 g protein/L resin, such as 5-50 g protein/L resin, typically 5 to 25 g protein/I resin, more typically 10-20 g protein/L resin. The column is washed with an aqueous solvent containing about 20-40 mmol/kg sodium caprylate, at a pH between about 5 and 6 for about 30 minutes after pH has stabilised. The column is optionally further washed with different solvents to remove impurities before the protein is eluted with a formic acid containing solvent.

The loading of a sample onto a stationary phase, may comprise one or more of the following chromatographic procedures: ion exchange chromatography, affinity chromatography, mixed-mode chromatography and hydrophobic interaction chromatography.

Accordingly, the stationary phase may comprise any chromotographic media known in the art, including an inert matrix fixed with a protein-binding ligand, a protein, an anion exchanger, a cation exchanger. Preferably, the stationary phase is a media for affinity chromatography.

The purification of the protein from its viral content or viral inactivation is an object of the invention. From Example 1, it is shown that the chromatographic step on a column with Capto L media and with caprylic acid wash completely clears the viral content, as determined by clearing of the spiked eMuLV. The complete clearance of eMuLV that was seen in Example 1 is also seen in Example 2 on a column with Protein A media. The purification of the protein from its viral content or viral inactivation as determined by the clearance of the viral load is thus not dependent on the media in the column.

When using recombinant techniques, the protein may be produced intracellularly, in the periplasmic space, or directly secreted into the sample medium. In one aspect, such as if the protein is produced intracellularly, prior to loading the sample on the solid phase, the particulate debris, either host cells or lysed cells (e.g., resulting from homogenization), may be optionally removed, e.g., by centrifugation or ultrafiltration. However, an advantage of the method is that such a preliminary step is not essential. Where the antibody is secreted into the medium, supernatants from such expression systems may be first concentrated using a commercially available protein concentration filter.

Where the protein is secreted into the medium, the recombinant host cells can also be separated from the cell culture medium, e.g., by tangential flow filtration. The protein of the invention can be purified from the culture medium using the method of the invention.

In one embodiment, the affinity chromatography step comprises subjecting the sample onto a column comprising a suitable affinity chromatographic support. Non- limiting examples of such chromatographic supports include, but are not limited to Protein A resin, Protein L resin, Protein G resin, affinity supports comprising the antigen against which the antibody of interest was raised, and affinity supports comprising an Fc binding protein. Protein A resin is useful for affinity purification and isolation of antibodies such as IgG.

Following the loading of the column, the column may be washed one or multiple times using, e.g., an equilibrating buffer. Other washes including washes employing different buffers can be used before eluting the column. The eluate can be monitored using techniques well known to those skilled in the art.

A suitable cation exchange column is a column whose stationary phase comprises anionic groups. An example of such a column is a SP Sepharose^{(TM)} column.

A suitable mixed -mode column is a column whose stationary phase comprises mixed-mode groups. An example of such a column is a Capto MMC ^{(TM)} column.

In another embodiment, the sample is subjected to hydrophobic interactive chromatography ("HIC"). A suitable column is one whose stationary phase comprises hydrophobic groups. An example of such a column is a phenyl Sepharose(TM) column. It is possible that the antibodies have formed aggregates during the isolation/purification process. This hydrophobic chromatographic step facilitates the elimination of these aggregations. It also assists in the removal of impurities.

According to the method of the invention, the protein-containing sample, such as cell line media or blood plasma is loaded onto a stationary phase, so as to bind the target protein to said stationary phase. The method of the invention is not limited to any stationary phase. The Examples demonstrate that different stationary phases can be used in the method of the invention. The chromatography steps can include one or more of the following chromatographic procedures: ion exchange chromatography, affinity chromatography, and hydrophobic interaction chromatography. The stationary phase is typically a part of a chromatography column. The stationary phase is typically a media for affinity chromatography. The stationary phase may be selected from an ion exchange chromatography column, a fast protein liquid chromatography, and an expanded bed adsorption (EBA) chromatography column, preferably an ion exchange chromatography column.

In one embodiment, the stationary phase comprises an agarose matrix fixed with an immunoglobulin-binding protein ligand, such as a recombinant protein or any protein which has a strong affinity to the variable region of antibody kappa light chains. An example of such a stationary phase is a column packed with Capto L media (GE-Healthcare). Accordingly, in one embodiment, the stationary phase comprises Protein L typically fixed to a resin. In a further embodiment, the stationary phase comprises a Protein A-derived affinity medium. An example of such a stationary phase is a column packed with Mab Select SuRe media. In a further embodiment, the stationary phase comprises an agarose matrixed with strong cation and/or strong anion exchangers. Examples of such stationary phases include Capto SP ImpRes and Capto Q ImpRes.

The duration of the exposure of the sample to the caprylic acid solution may vary with a number of factors, including, but without being limited to, concentration of free caprylic acid, pH, type of virus and temperature. Table A indicates pH required at various concentrations of caprylate to obtain preferred exposure times of samples to the caprylic acid solution. For most practical purpose, step ii. subjecting the solid phase to a caprylic acid solution, or subjecting the sample to a caprylic acid solution takes place for less than 2 hours, typically less than 1 hour, such as for 0.05 minutes to 50 minutes, more typically from 0.10 minutes to 30 minutes, preferably 0.25 minutes to 15 minutes.
As can be seen from Tables 6 and 7, the viral in activation is often achieved in less than 5 minutes. However, depending on the conditions (such as concentration of free caprylic acid, pH, type of virus and temperature) required up to 1 hour, such as up 45 minutes, such as 30 minutes, for practical purposes.

In a typical embodiment of the invention, a column packed with a resin and loaded the target protein is washed with an aqueous solvent containing caprylic acid for about 30 minutes. The column is optionally further washed with different solvents to remove impurities before the protein is eluted with a solvent that removes the target protein.

The caprylic acid solution has a pH so as to inactivate the virus and not denature the target protein. The caprylic acid solution has a pH so as to form a non-ionic or free caprylic acid. Accordingly, the pH of the caprylic acid solution is typically acidic.

Accordingly, the caprylic acid solution has a pH of 7.0 or less, such as a pH between 2 and 7, such as less than 6.5, such as less than 6.1, such as a pH from 2-6, such as at a pH of 2<pH≤ 6, preferably 3<pH≤ 6, more preferably 4<pH≤ 6, such as 4.5<pH≤ 6, most preferably 2<pH<6, preferably 3<pH< 6, more preferably 4<pH< 6, such as 4.5<pH<6, such as 4.6 ≤pH<6, such as about 4.6, about 4.7, about 4.8, about 4.9, about 5.0, about 5.1, about 5.2, about 5.3, about 5.4, about 5.5, about 5.6, about 5.7, about 5.8, and about 5.9, such as 4.9<pH<6.

As can be seen from Example 2, low concentrations of free caprylic acid are still effective for complete inactivation of a virus such as eMuLV. In a preferred embodiment of the present invention, the caprylic acid itself is the buffer (pKa = 4.9). Typically, the caprylic acid solution is a caprylic acid buffer at a concentration of 1 to 100 mM, typically 1 to 50 mM, including 10 to 80 mM , such as 30 to 70 mM or 30 to 50 mM. Preferably, the concentration of non-ionised caprylic acid is 1 to 10 mM, such as 2 to 10 mM, such as 2 to 8 mM , such as 2 to 6 mM. In a typical embodiment, the buffer used is made by combining 35 mmol/kg Na-caprylate with 4.1 mmol/kg HCI, resulting in a pH of about 5.6-6.7. The concentration of non-ionised caprylic acid may be determined by the Henderson-Hasselbach equation: pH = pKa + log([caprylate]/[caprylic acid]).

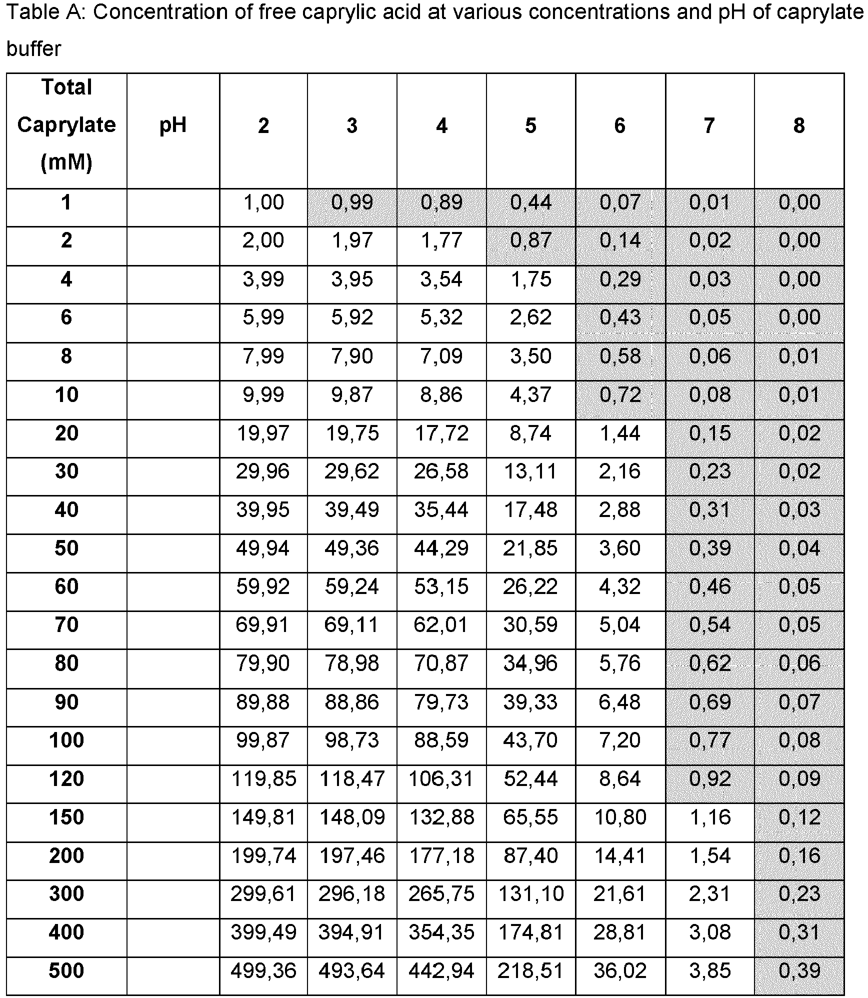

Table A shows the pH required at various concentrations of caprylate, or conversely the concentration required at various pH levels, in order to achieve the required minimum concentration of 1 mM of free caprylic acid for the protein purification of the present invention. The shaded area delineates embodiments wherein the concentration of free caprylic acid is too low for practical purposes in that viral inactivation would require a too long exposure time in order to inactivate a acceptable amount of viruses according to the present invention.

The caprylic acid solution is typically a caprylic acid buffer at a concentration of 1 to 500 mM, typically 1 to 50 mM, including 1 to 20 mM, such as 2 to 20 mM or 1 to 10 mM, more preferably 2 to 10 mM such as 2 to 8 mM, such as 2 to 6 mM, as measured in terms of free caprylic acid.

The caprylic acid solution typically comprises a combination i) a caprylate salt and ii) an inorganic or organic acid in proportions so provide a solution of pH less than 7.0, such as 6.5 or less, such as from pH 1 to 6.2, and in a concentration of caprylic acid at a concentration of at least 1 mM, such as at least 2 mM, such as from 2 to 100 mM, such as from 2.5 to 100 mM, as measured in terms of free caprylic acid.

The loading of the protein-containing sample and/or the washing with the caprylic acid buffer can be done at a diversity of temperatures, typically from 1 to 40 °C, such as from 1 to 30 °C, such as 2 to 25 °C. The low temperature used in Example 4, compared to the temperature used in example 3, shows the viral inactivation step is achieved at both low temperatures (2 to 8 °C) and at higher temperatures (15 to 25 °C)

Elution of the protein may be done by conventional techniques such as an elution buffer, such as an acidic elution buffer, typically using an organic acid. In a preferred embodiment, the elution buffer is formic acid. In a suitable embodiment the elution buffer is prepared by combining formic acid 10 mmol/kg plus NaOH 3.4 mmol/kg, resulting in a pH of approx. 3.5). The elution step can also be performed by a buffer with high salt.

In a typical embodiment of the invention, a column packed with a resin is loaded with clarified culture broth containing the target protein; the column is typically loaded to approximately 10-40 g protein/L resin, such as 20-40g protein/L resin, and the column is washed with an aqueous solvent containing about 20-40 mmol/kg sodium caprylate, at 2 to 25 °C, at a pH between about 5 and 6 for about 30 minutes after pH has stabilised. The column is optionally further washed with different solvents to remove impurities before the protein is eluted with a formic acid containing solvent.

The invention is furthermore directed to a protein, in solid or solution form, obtained by the method of the invention. In yet another embodiment, the invention is directed to one or more pharmaceutical compositions comprising a protein, such as an isolated monoclonal antibody or antigen-binding portion thereof obtained by the method of the invention and an acceptable carrier.

The following non-limiting examples are provided for illustrative purposes only in order to facilitate a more complete understanding of representative embodiments now contemplated. These examples should not be construed to limit any of the embodiments described in the present specification, including those pertaining to the methods of inactivating a lipid-coat containing virus disclosed herein and products processed using these methods.
Without limitation, the following embodiments are preferred modes of the invention.
1. A method for the purification of a target protein from a sample comprising the steps of
   i. loading a protein-containing sample onto a stationary phase, so as to bind the target protein to said stationary phase;
   ii. subjecting the solid phase with the bound target protein to a caprylic acid solution; wherein the caprylic acid solution is at a pH so as to form a free caprylic acid, and wherein the caprylic acid solution is a caprylic acid buffer at a concentration of 1 to 50 mM, as measured in terms of free caprylic acid.and
   iii. eluting the target protein
2. A method, according to embodiment 1, wherein purification of a target protein comprises inactivation of virus in said sample.
3. A method according to any one of embodiments 1 or 2 for the preparation of a virus-free protein solution.
4. The method according to embodiments 1 to 3, wherein the caprylic acid solution comprises a combination i) a caprylate salt and ii) an inorganic or organic acid in proportions so provide an acidic solution and a concentration of caprylic acid greater than 1 mM, such as at least 2 mM, such as at least 2.2 mM, typically at least 2.5 mM, as measured in terms of free caprylic acid.
4. The method according to embodiments 1 to 3, wherein the caprylic acid solution comprises a combination i) a caprylate salt and ii) an inorganic or organic acid in proportions so provide a solution of pH less than 7.0, such as 6.5 or less, and in a concentration of caprylic acid at a concentration of at least 1 mM, such as at least 2 mM, such as from 2 to 10 mM, as measured in terms of free caprylic acid.
5. A method according to embodiments 1 to 4, wherein the protein is selected from the group consisting of an antibody, or a fragment thereof, a coagulation factor, such as FV, FVII, FVIII, FIX, FX, FXI, and FVIII, and a growth hormone.
6. A method according to any of the preceding embodiments wherein the sample is selected from the group consisting of a fermentation broth, a cell culture, cell line media, ascites fluid, tissue culture media, a transgenic cell medium, human or animal plasma including plasma fractions.
7. A method according to any the preceding embodiments wherein the stationary phase is a chromatography column.
8. A method according to embodiment 7 wherein the sample is loaded onto a stationary phase selected from an ion exchange chromatography column, an affinity chromatography, a mixed-mode chromatography, a fast protein liquid chromatography, and an expanded bed adsorption (EBA) chromatography column.
9. A method according to any of the preceding embodiments wherein the caprylic acid solution has a pH so as to inactivate the virus and not denature the target protein.
10. A method according to any of the preceding embodiments wherein caprylic acid solution has a pH of 6.1 or less, such as 2-6, such as at a pH of 2<pH≤ 6, preferably 3<pH≤ 6, more preferably 4<pH≤ 6, such as 4.5<pH≤ 6, , such as 4.6 ≤pH<6, such as about 4.6, about 4.7, about 4.8, about 4.9, about 5.0, about 5.1, about 5.2, about 5.3, about 5.4, about 5.5, about 5.6, about 5.7, about 5.8, and about 5.9, such as 4.9<pH<6.
11. A method according to any of the preceding embodiments wherein caprylic acid solution is a caprylic acid buffer of at a concentration of 1 to 20 mm, such as 2 to 20 mm or 1 to 10 mm, more preferably 2 to 10 mm such as 2 to 8 mm, such as 2 to 6 mm, as measured in terms of free caprylic acid.
12. A method according to any of the preceding embodiments wherein the sample comprises enveloped viruses, non-enveloped virus or both non-enveloped and enveloped viruses.
13. A method according to any of the preceding embodiments, which is automated or part of an automated protein purification process.
14. A method according to any of the preceding embodiments, which further inactivates mycoplasma.
15. A method for the purification of proteins selected from a coagulation factor, an antibody or a Fab thereof comprising the steps of
   i. loading a sample comprising one or more of said proteins onto a stationary phase for affinity chromatography;
   ii. subjecting to solid phase to a 2 to 10 mM such as 2 to 8 mM , such as 2 to 6 mM, of caprylic acid solution, as measured in terms of free caprylic acid at a pH of 4.9<pH<6.2, so as to inactivate and wash away viruses;
   iii. eluting the target protein.

A further advantage of the method of the invention is that is provides a virus inactivated sample, or a sample free of viruses and furthermore a sample wherein mycoplasmas are inactivated. Furthermore, the method of the invention provides samples with a reduced amount of impurities such as Host Cell Protein (HCP).

### EXAMPLES

### Example 1

A cell line media containing a recombinant human Fab-fragment was filtered through a 0.22 µm and then spiked with eMuLV virus. The spiked media was applied to a column packed with Capto L media (GE-Healthcare). After loading, the column was first washed with equilibration buffer (20 mM phosphate, 150 mM NaCl, pH 7.2), and then with a 35 mM caprylic acid buffer at pH 5.5 (5.8 mM free caprylic acid). After washing with the caprylic acid buffer for 30 minutes, the Fab-fragment was eluted from the column with a 20 mM formic acid at pH 3.5. Process temperature was 15-25 °C. The yield of Fab-fragment was 97 %, and no eMuLV was detected in the eluted fraction containing the Fab-fragment (Table 1).

**Table 1**

| Sample | Total virus, PFU/ml | LRV |
|---|---|---|
| Load | 1.5 × 10⁶ | >6.0 |
| Fab-fragment fraction | <1.5* | |

| | | |
|---|---|---|
| * Detection limit of the assay | | |

This shows that the chromatographic step on a column with Capto L media and with caprylic acid wash, completely clears the spiked eMuLV with a LRV of more than 6.0 log₁₀.

### Example 2

A cell line media containing a recombinant human antibody (IgG₁) was filtered through a 0.22 µm filter and spiked with eMuLV virus. The spiked media was loaded on a column packed with Protein A (Mab Select SuRe, Ge Healthcare). After loading, the column was first washed with equilibration buffer (50 mmol/kg phosphate, 300 mmol/kg NaCl, pH 7.0), and then with a 32 mmol/kg caprylate buffer, pH 5.8 (2.9 mM free caprylic acid). Low concentration of caprylate and high pH was used to show effectiveness of this step at worst-case conditions (low caprylic acid concentration). After pH had stabilised the washing continued for 30 min. After 3 other washings (equilibration buffer, 50 mmol/kg phosphate, 1 mol/kg NaCl and then equilibration buffer), the mAb was eluted from the column with a formate buffer, pH 3.5. The yield of the mAb was 100%, and no eMuLV was detected in the eluted fraction containing the mAb (Table 2). The process temperature was 15-25 °C.

**Table 2**

| Sample | Total virus, PFU/ml | LRV |
|---|---|---|
| Load | 9.20 × 10⁴ | >3.0 |
| mAb fraction | <1.0 × 10²* | |

| | | |
|---|---|---|
| * Detection limit of the assay | | |

The complete clearance of eMuLV that was seen in example 1, is also seen on a column with Protein A media. The clearance of the spiked load is thus not dependent on the media in the column. Also, low concentrations of free caprylic acid are still effective for complete clearance of eMuLV.

### Example 3

A cell line media containing a recombinant human antibody (IgG₄) was filtered through a 0.22 µm filter and spiked with eMuLV virus. The spiked media was loaded on a column packed with Protein A (Mab Select SuRe, Ge Healthcare). After loading, the column was first washed with equilibration buffer (50 mmol/kg phosphate, 300 mmol/kg NaCl, pH 7.0), and then with a 32 mmol/kg caprylate buffer, pH 5.8 (2.9 mM free caprylic acid). After pH had stabilised the washing continued for 30 min. After 3 other washing (equilibration buffer, 50 mmol/kg phosphate, 1 mol/kg NaCl) and then equilibration buffer), the mAb was eluted from the column with a formate buffer, pH 3.5. The yield of the mAb was 90 %, and no eMuLV was detected in the eluted fraction containing the mAb (Table 3). The process temperature was 15-25 °C.

**Table 3**

| Sample | Total virus, PFU/ml | LRV |
|---|---|---|
| Load | 7.40 × 10⁴ | >2.8 |
| mAb fraction | <1.2 × 10²* | |

| | | |
|---|---|---|
| * Detection limit of the assay | | |

This example with another human antibody as in example 2 shows that the complete clearance of eMuLV is independent of the monoclonal antibody in the cell line media.

### Example 4

A cell line media containing a recombinant human antibody (IgG₁) was filtered through a 0.22 µm filter and spiked with eMuLV virus. The spiked media was loaded on a column packed with Protein A (Mab Select SuRe, Ge Healthcare). After loading, the column was first washed with equilibration buffer (50 mmol/kg phosphate, 300 mmol/kg NaCl, pH 7.0), and then with a 35 mmol/kg caprylate buffer, pH 5.7 (3.9 mM free caprylic acid). After pH had stabilised the washing continued for 30 min. After 3 other washings (equilibration buffer, 50 mmol/kg phosphate, 1 mol/kg NaCl and then equilibration buffer), the mAb was eluted from the column with a formate buffer, pH 3.5.

The yield of the mAb was 90 %, and no eMuLV was detected in the eluted fraction containing the mAb (Table 4). The process temperature was 2-8 °C.

**Table 4**

| Sample | Total virus, PFU/ml | LRV |
|---|---|---|
| Load | 8.82 × 10⁴ | >3.0 |
| mAb fraction | <9.0 × 10¹* | |

| | | |
|---|---|---|
| * Detection limit of the assay | | |

The low temperature used in this example, compared to the temperature used in example 3, shows that a total clearance of eMuLV is achieved at both low temperatures (2 - 8 °C) and at higher temperatures.

### Example 5

170 litres of a cell line media containing a recombinant human antibody (IgG₁) was in the pilot plant divided into two equal parts and then run separately through a column with Protein A (Mab Select SuRe, Ge Healthcare) on an automatic chromatography system (ÄKTA pilot, GE Healthcare).

One part was loaded to a Protein A column (7.1 litres of Mab Select SuRe, Ge Healthcare), where after the column was washed with 3 buffers (equilibration buffer, (50 mmol/kg phosphate, 1 mol/kg NaCl) and then equilibration buffer). The mAb was eluted from the column with a formate buffer, pH 3.5. The eluate was manually treated at low pH (3.7) for 60 minutes.

The other part was handled as in example 4 with a programmed caprylic acid wash step during the Protein A chromatography (7.1 litres of Mab Select SuRe, Ge Healthcare) and no treatment of the eluate at low pH.

The eluates were then separately loaded to a cation exchange column (2.2 litres of Poros 50 HS, Life Technologies). After washing of the columns with equilibration buffer (37.4 mM acetic acid buffer, pH 5), the columns were eluted with a linear gradient from 0 to 300 mM NaCl in acetic acid buffer, pH 5).

The level of HMWP was about 2 % in both eluates, but increased to 3 % after low pH treatment (see table). After cation exchange the level of HMWP was about 2 % in both the part that was treated with low pH and the part that was treated with a caprylic acid wash during Protein A chromatography. The level of CHO-HCP was only about half the level in the eluate from the Protein A column that was washed with caprylic acid, compared to the level in the eluate that was treated with low pH (see table). After cation exchange, the level of CHO-HCP in the part that was treated with a caprylic acid wash during Protein A chromatography was still half the amount of CHO-HCP in the part that was treated with the low pH.

The process with caprylic acid wash during Protein A chromatography was about 45 minutes faster than the process with low pH treatment of the elute from Protein A. The yield of monoclonal antibody was almost the same in the two parts after cation exchange (Table 5).

**Table 5**

| Step | Assay | Low pH treatment after Protein A | Caprylic acid wash during Protein A |
|---|---|---|---|
| Eluate from Protein A | HMWP, % | 2.2 | 2.1 |
| | CHO-HCP, ng/ml | 13918 | 6531 |
| After pH treatment | HWMP, % | 3.0 | N/A* |
| Eluate from cation exchange | HMWP, % | 2.1 | 2.1 |
| | CHO-HCP, ng/ml | 1371 | 601 |
| | Amount of antibody, g | 142 | 156 |

| | | | |
|---|---|---|---|
| * N/A: Low pH treatment was not performed | | | |

The example shows that a lower concentration of CHO-HCP is achieved by using a caprylic acid wash step during chromatography on a column with Protein A. The lower amount of CHO-HCP is still seen after the next chromatographic step (cation exchange). Also, the increase of HMWP after the low-pH step is avoided. 45 minutes in process time was saved be using the process with a caprylic acid wash compared to the process with low-pH step.

### Example 6

The inactivation of MuLV was investigated at different concentrations of caprylic and at different pH (Table 6). In the pH range of 5.0 to 6.0 was achieved complete inactivation of MuLV after 5 minutes if caprylic acid concentrations were raised while the pH was increased, so that the free caprylic acid concentration was maintained at at least about 4 mM. Reaction times were increased at caprylic acid concentrations of about 2 mM, with partial inactivation with these most difficult viruses after 30 minutes. Below 1mM, no inactivation was observed.

**Table 6: Titre of MuLV virus after inactivation with caprylic acid at differing concentrations and pH**

| Caprylic acid + caprylate | Free caprylic acid mM | MuLV content | 1 minute | 5 minutes | 10 minutes | 30 minutes |
|---|---|---|---|---|---|---|
| 10 mM, pH 5,0 | 4,4 | 1,2 × 10⁶ | 5,0 × 10¹ | 0 | 0 | 0 |
| 20 mM, pH 5,5 | 3,9 | 1,2 × 10⁶ | 2,5 × 10² | 0 | 0 | 0 |
| 60 mM, pH 6,0 | 4,3 | 1,2 × 10⁶ | 0 | 0 | 0 | 0 |
| 30 mM, pH 6,0 | 2,2 | 1,2 × 10⁶ | >5 × 10⁴ * | >5 × 10⁴ | >5 × 10⁴ | 3,1 × 10⁴ |
| 10 mM, pH 6,0 | 0,7 | 1,2 × 10⁶ | >5 × 10⁴ | >5 × 10⁴ | >5 × 10⁴ | >5 × 10⁴ |

| | | | | | | |
|---|---|---|---|---|---|---|
| * To many virus plaques to be counted | | | | | | |

The concentration of caprylic acid depends on the pH, and it is only the free captylic acid that inactivate enveloped viruses. The efficiency of 60 mM caprylic acid was therefore further investigated in the pH range 5.9 to 6.3. Throughout this pH range, there was a complete inactivation of more than 5.4 log10 of MuLV within 5 minutes (Table 7). At pH 6.3, the caprylic acid concentration is 2.2 mM, and this concentration is also effective in this test.

With a total concentration of caprylic acid at 60 mM, inactivation of MuLV was highly effective at pH ranging from 5.9 to 6.2. Best results were obtained when the pH was maintained at 6.1 or less with 60 mM caprylic.

**Table 7: Titre of MuLV after inactivating of MuLV with 60 mM caprylic acid at different pH levels**

| pH | Free caprylic acid mM | MuLV content | 1 minute | 5 minutes | 10 minutes | 30 minutes |
|---|---|---|---|---|---|---|
| 5,9 | 5,3 | 2,7 × 10⁵ | 2,5 × 10¹ | 0 | 0 | 0 |
| 6,0 | 4,3 | 2,7 × 10⁵ | 2,5 | 0 | 0 | 0 |
| 6,1 | 3,5 | 2,7 × 10⁵ | 0 | 0 | 0 | 0 |
| 6,2 | 2,8 | 2,7 × 10⁵ | 2,3 × 10² | 0 | 0 | 0 |
| 6,3 | 2,2 | 2,7 × 10⁵ | 1,3 × 10¹ | 0 | 0 | 0 |

To be effective, it is shown that the concentration of free caprylic acid should be at least 1 mM, preferably at least about 2 mM, such as about 2,2 mM, such as at least about 2.5 mM or at least 2.8 mM.

As the person skilled in the art will readily recognize, a high concentration of other salts, such as CaCl₂ or NaCl, is likely to reduce the solubility of caprylic acid. As the person skilled in the art will readily recognize, concentrations of non-caprylate salts of more than 500 mM, such as more than 100 mM or more than 50 mM, should be avoided as this may impact the ability of free caprylic acid to be generated. However, the person skilled in the art will readily recognize that the concentration of caprylic acid should be no higher than its solubility.

A protein purification process comprising viral inactivation using caprylic acid has been shown to be very effective and quick in the inactivation of enveloped viruses in general, including MuLV virus, at concentrations of free caprylic acid of above 1 mM. While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A method for the purification of a target protein from a sample comprising the steps of
i. loading a protein-containing sample onto a stationary phase, so as to bind the target protein to said stationary phase;
ii. subjecting the solid phase with the bound target protein to a caprylic acid solution; wherein the caprylic acid solution is at a pH so as to form free caprylic acid, and wherein the caprylic acid solution is a caprylic acid buffer at a concentration of 1 to 50 mM, as measured in terms of free caprylic acid; and
iii. eluting the target protein

2. A method, according to claim 1, wherein purification of a target protein comprises inactivation of virus in said sample.

3. A method according to any one of claims 1 or 2 for the preparation of a virus-free protein solution.

4. The method according to claims 1 to 3, wherein the caprylic acid solution comprises a combination i) a caprylate salt and ii) an inorganic or organic acid in proportions so provide a solution of pH less than 7.0, such as 6.5 or less, and in a concentration of caprylic acid at a concentration of at least 1 mM, such as at least 2 mM, such as from 2 to 10 mM, as measured in terms of free caprylic acid.

5. A method according to claims 1 to 4, wherein the protein is selected from the group consisting of an antibody, or a fragment thereof, a coagulation factor, such as FV, FVII, FVIII, FIX, FX, FXI, and FVIII, and a growth hormone.

6. A method according to any of the preceding claims wherein the sample is selected from the group consisting of a fermentation broth, a cell culture, cell line media, ascites fluid, tissue culture media, a transgenic cell medium, human or animal plasma including plasma fractions.

7. A method according to any of the preceding claims, wherein the a stationary phase is a chromatography column.

8. A method according to claim 7 wherein the sample is loaded onto a stationary phase selected from an ion exchange chromatography column, an affinity chromatography, a mixed-mode chromatography, a fast protein liquid chromatography, and an expanded bed adsorption (EBA) chromatography column.

9. A method according to any of the preceding claims wherein the caprylic acid solution has a pH so as to inactivate the virus and not denature the target protein.

10. A method according to any of the preceding claims wherein caprylic acid solution has a pH of 6.1 or less, such as 2-6, such as at a pH of 2<pH≤ 6, preferably 3<pH≤ 6, more preferably 4<pH≤ 6, such as 4.5<pH≤ 6, , such as 4.6 ≤pH<6, such as about 4.6, about 4.7, about 4.8, about 4.9, about 5.0, about 5.1, about 5.2, about 5.3, about 5.4, about 5.5, about 5.6, about 5.7, about 5.8, and about 5.9, such as 4.9<pH<6.

11. A method according to any of the preceding claims wherein caprylic acid solution is a caprylic acid buffer of at a concentration of 1 to 20 mM, such as 2 to 20 mM or 1 to 10 mM, more preferably 2 to 10 mM such as 2 to 8 mM, such as 2 to 6 mM, as measured in terms of free caprylic acid.

12. A method according to any of the preceding claims wherein the sample comprises enveloped viruses, non-enveloped virus or both non-enveloped and enveloped viruses.

13. A method according to any of the preceding claims, which is automated or part of an automated protein purification process.

14. A method according to any of the preceding claims, which further inactivates mycoplasma.

15. A method for the purification of proteins selected from a coagulation factor, an antibody or a Fab thereof comprising the steps of
i. loading a sample comprising one or more of said proteins onto a stationary phase for affinity chromatography;
ii. subjecting to solid phase to a 2 to 10 mM such as 2 to 8 mM , such as 2 to 6 mM, of caprylic acid solution, as measured in terms of free caprylic acid at a pH of 4.9<pH<6.2, so as to inactivate and wash away viruses;
iii. eluting the target protein.
